# EUROPEAN PATENT SPECIFICATION

(11) **EP 1 585 003 B1**
(45) Date of publication and mention of the grant of the patent: **30.01.2008**
(21) Application number: 04425252.6
(22) Date of filing: 05.04.2004
(51) Int. Cl.: G05D 16/04, A61M 16/00, A62B 7/00, F23N 1/00

(54) **Reduction pressure device for a gas cylinder**
Druckreduziervorrichtung für Gasflasche
Dispositif de réduction de pression d'un cylindre à gaz

(43) Date of publication of application: 12.10.2005
(73) Proprietor: Flow Meter S.p.a., 24040 Levate (Bergamo) (IT)
(72) Inventor: Paratico, Roberto c/o Flow Meter S.p.A., 24040 Levate (BG) (IT)
(74) Representative: Botti, Mario

(56) References cited:
- GB-A- 854 446
- US-A- 3 915 378
- US-A- 4 722 333

## Description

### Field of Application

The present invention relates to a pressure reduction device for a gas cylinder.

In particular, the device according to the invention is of the type comprising a valve body intended for being co-axially associated with the gas cylinder and a gas path extended in the valve body between a gas inlet at high pressure and a gas outlet at a reduced pressure. The device according to the invention also comprises gas interception means, at least one pressure reduction valve and a flow-rate regulation valve, placed along the gas path.

Preferably, but not exclusively, the device according to the invention is applicable to gas cylinders for medical use, for example for the delivery of oxygen for respiratory therapies.

### Prior Art

It is known the need of providing pressure reduction devices of the above specified type being mounted at the inlet of a gas cylinder, which allow to selectively deliver the gas from the cylinder by opening/closing an interception valve, besides ensuring the tight closing of this interception valve, also in case of high pressure of the inputting gas.

In general, the known devices provide the opening and the closing of the delivery by means of the rotation of a knob directly connected with a cut-off with axial movement with respect to the gas path. An interception valve of the type mentioned above is known for example from US patent 4,722,333.

In particular, a pressure reduction device has been proposed suitable for being co-axially associated with the gas cylinder, which provides the use of an interception valve being axially movable with respect to the main axis of the device.

In particular, the known device provides a valve body divided into a first block, which comprises a pressure reduction valve and a flow-rate regulation valve, and in a second block which comprises the gas interception valve.

The known device further comprises a rotary tubular knob, co-axially associated with the valve body, which is provided with a cam profile for co-operating with a corresponding profile made in the first block.

For opening/closing the interceptions valve, the knob is rotated around its axis, in order to axially move the first block by means of the cam profile. The first block axially moves in turn the interception valve by means of a rod, causing the opening or closing thereof.

Another pressure reduction device for a gas cylinder is also known, which, as the one previously described, comprises a rotary tubular knob, co-axial with the valve body, for controlling the interception valve.

In this case, the interception valve comprises a drawer, which is arranged in a housing made in the valve body and is transversally movable with respect to the axis of the valve body. The drawer ends project from the valve body and they co-operate with the guiding curved surfaces, made on the inner wall of the tubular knob.

In this way, by rotating the tubular knob, a transversal shift of the drawer is obtained, and thus the opening/closing of the interception valve.

The known pressure reduction devices, although allowing to selectively deliver the gas and to ensure the gas tightness when the interception valve is close, have however the drawback that the whole actuation mechanism for opening/closing this valve is rather articulated and complex, and it requires specific mechanical precise manufacturing for realising the single components, with minimum admitted tolerances and unavoidable high costs to be afforded for possible substitutions or components repairs.

The technical problem underlying the present invention is therefore that of realising a pressure reduction device for a gas cylinder which comprises an interception valve, which, besides allowing a selective delivery of the gas from the cylinder, allows to overcome the above drawbacks and which comprises components of simple realisation, which do not necessarily require mechanical manufacturing of precise thrust, and which are further easy to be assembled and which can be substituted in case of damage or for the upkeep of the apparatus.

### Summary of the invention

The technical problem is solved by a pressure reduction device of the above specified type wherein, according the present invention, as defiled in annexed claim 1.

### Brief description of the drawings

Further characteristics and advantages of the invention will be apparent from the following description of an embodiment, given by way of indicative, non-limiting example with reference to the annexed drawings.
Figure 1 shows a view of a pressure reduction device according to the invention.
Figure 2 is a section of the device of figure 1.
Figure 3 shows an enlarged detail of the device of figure 1.
Figure 4 shows a top view of an enlarged detail of the device of figure 1.

### Detailed description

With reference to the annexed drawings, the reference number 10 indicates a pressure reduction device for a pressurised gas cylinder, not shown in the drawings, preferably but not exclusively a gas cylinder for medical use.

For example, in the specific case, the pressure of the gas in the cylinder can reach values equal to 200 bar.

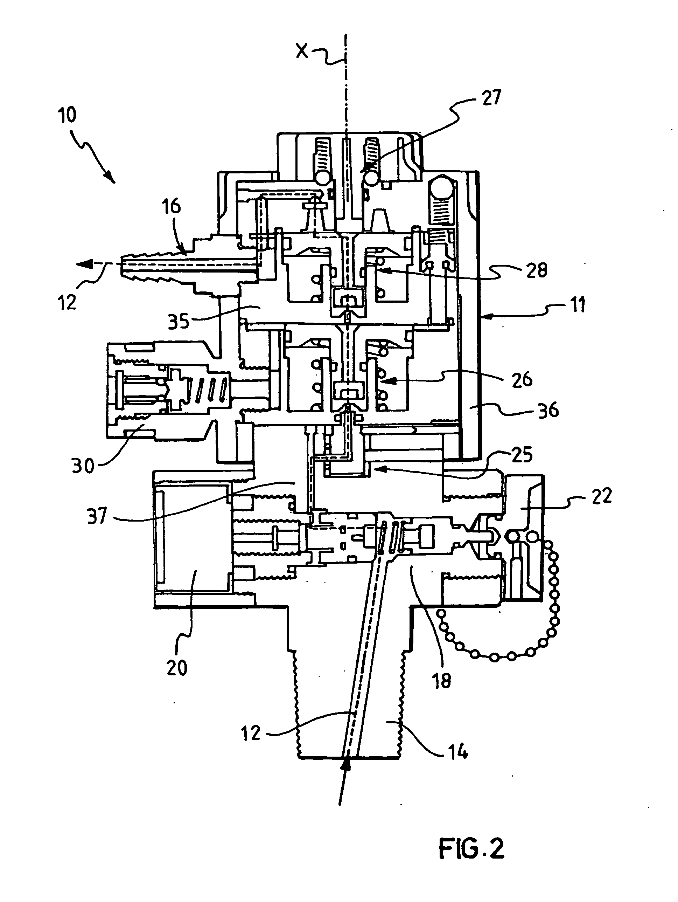

The device 10 is of the type comprising a valve body 11, intended for being associated, co-axially along a reference axis X (figure 2), with a gas cylinder, not shown in the drawings.

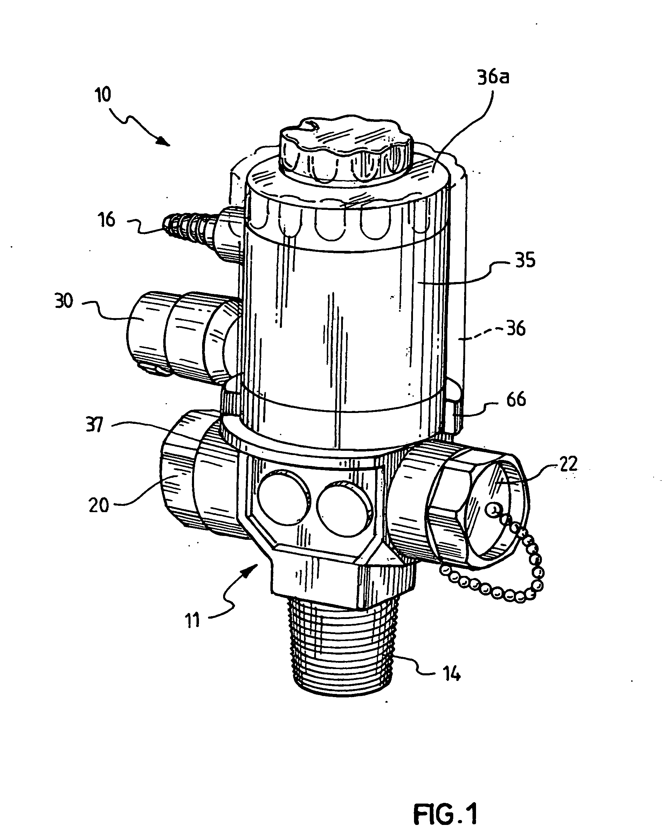

The valve body 11 (figure 1 and 2) substantially comprises three blocks, i.e. a lower support element 18, an intermediate body 37 and an upper containing structure 35.

In the shown solution, the intermediate body 37 of the valve body 11 is preferably integrally made with the support element 18.

The support element 18 is further provided with a gauge 20 for the visual control by the user of the pressure level of the gas contained in the cylinder, and a joint 22 which can be connected with a vessel for filling the gas cylinder, not shown in the drawings, by means of a flexible tube suitable for high pressure, not shown in the drawings either.

The device 10 further comprises a gas path 12, indicated with a dotted line in figure 2, which is extended in the valve body 11 between an inlet 14 of the gas at high pressure, and an outlet 16 of the gas at reduced pressure.

In particular, the gas inlet 14 is made in the support element 18, whereas the gas outlet 16 is associated with the containing structure 35.

The device 10 according to the invention further comprises three valves placed along the gas path 12.

The first valve is an interception valve 25 of the gas path 12; the second valve is a gas pressure reduction valve 26, also called first stage regulator wherein the pressure is reduced for example from the pressure of 200 bar to the pressure of around 4 bar, i.e. to the operation pressure of the systems for delivering medical gases, the third valve is a flow-rate regulation valve 27 of the gas.

Preferably, but not exclusively, the device 10 further comprises a second gas pressure reduction valve 28, also called second stage regulator, interposed between the outlet 16 and the flow-rate regulation valve 27. The second reduction valve 28 determines a pressure reduction from around 4 bar to around 1,9 bar and it allows a further gas pressure reduction necessary to guarantee a constant pressure value on the gauged orifices of the flow-rate regulation valve 27, and as a consequence a constant and accurate value of the delivered flow, independently from the pressure value of the gas contained in the cylinder.

The pressure reduction valve 26 is also in communication with a further outlet 30 of gas at medium pressure.

The first and the second pressure reduction valves 26, 28 and the gas flow-rate regulation valve 27 are integrated inside the containing structure 35. These valves are of the known type and therefore they are not further described hereafter in the text.

The device 10 further preferably comprises a tubular knob 36, shown in transparency in figure 1, which is substantially arranged co-axially to the valve body 11, and it is angularly movable around the axis X. In particular, the knob 36 is peripherally mounted around the containing structure 35 and to the intermediate body 37. The function of the knob 36 is described in deeper detail hereafter in the description.

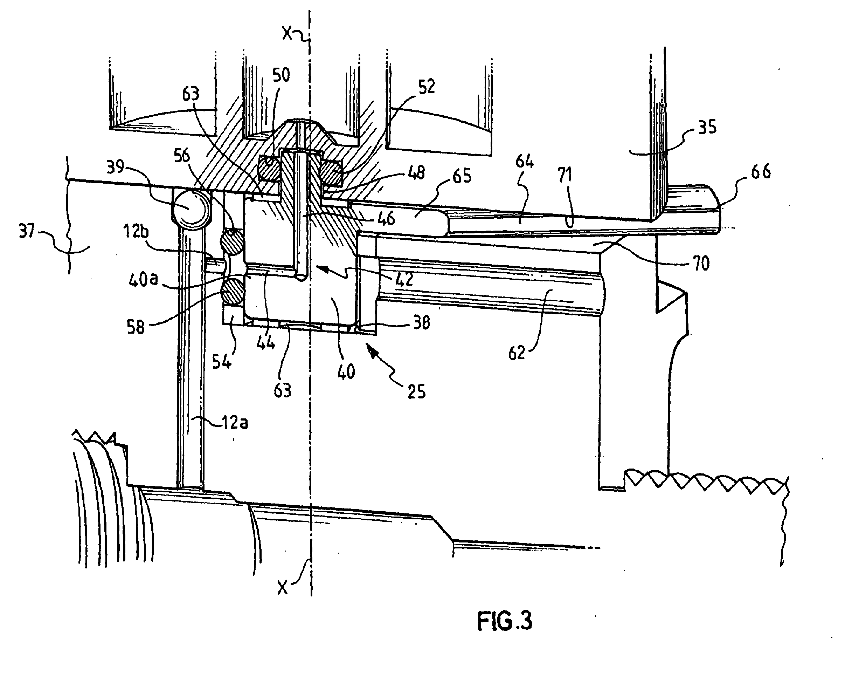

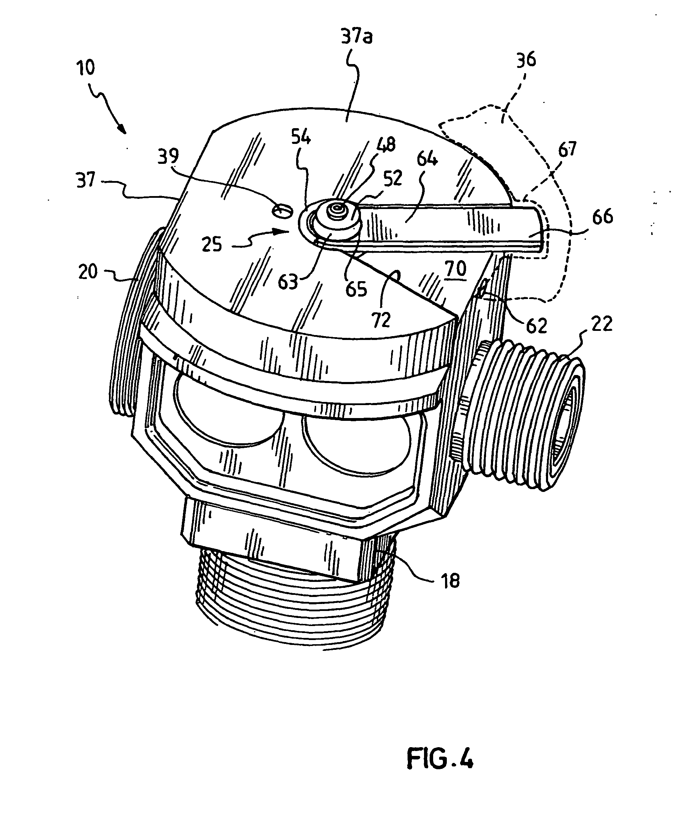

According to the present invention, the gas interception valve comprises a rotary valve (figures 3 and 4). This latter preferably comprises a seat 38 made in the intermediate body 37, preferably of cylindrical shape, and put in communication with the gas path 12.

In the specific case, the seat 38 is in communication with a substantially horizontal section 12b of the gas path 12, which is connected with a vertical section 12a. The upper end of the vertical section 12a is closed to the gas passage by means of a tight gasket 39, substantially with circular sector, fixed in the intermediate body 37.

The rotary valve 25 also comprises a rotary element 40, or male, rotary around the axis X, which is housed in the seat 38 and which can be angularly shifted for selectively opening/closing the gas path 12.

The rotary element 40 is provided with a gas passage 42, which is preferably L-shaped, and which comprises a radial section 44, substantially placed at the height of the horizontal section 12b of the path 12 and an axial section 46.

Preferably, the rotary element 40 is a full cylindrical body having a closed side wall 40a and the gas passage 42 is made inside the cylindrical body.

The axial section 46 of the gas passage 42 further extends in an appendix 48 integrally realised with the rotary element 40. The appendix 48 is inserted in a corresponding hollow 50 which is made in the overhanging cylindrical structure 35 and which is tightly closed by a first ring 52.

According to another characteristic of the present invention, the rotary valve 25 comprises a jacket 54, which is inserted in the intermediate body 37 between the seat 38 and the rotary element 40.

The jacket 54 has a substantially cylindrical shape and it is made of anti-friction metal, preferably of bronze, with a bearing function of support of the angular shift of the rotary element 40 and of protection from wear of this latter.

The valve 25 further comprises a radial opening 56 made in the jacket 54, in correspondence with the horizontal section 12b of the gas path 12, and a second tight ring 58 which is inserted in the radial opening 56 of the jacket 54.

The jacket 54 allows to maintain the second tight ring 58 firmly in its position.

From the opposite part with respect to the tight ring 58, the rotary valve 25 comprises a second opening, not visible in the drawings, which is arch-shaped. The second opening is coupled with a pivot 62, pressed in the body valve 11, and it is necessary for guaranteeing a lock in position, , and for avoiding the rotation of the jacket 54 together with the second tight ring 58 during the angular movement of the rotary element 40.

Washers 63 are also interposed with spacing functions for the exact axial placement of the rotary element 40 in the seat 38 and for guaranteeing an anti-friction bearing deriving from the axial load of the rotary element 40 due to the pressure of the gas in the cylinder.

According to a further characteristic of the present invention, the rotary valve 25 comprises a manoeuvre arm 64, flat-shaped and having rectangular section, which radially extends from the rotary element 40 outwards from the intermediate body 37.

The manoeuvre arm 64 has an end 65 being integral with the rotary element 40 and it comprises, at the opposite end, a terminal section 66 projecting from the intermediate body 37 and suitable for co-operating with the above cited knob 36 for activating the angular shift of the rotary element 40.

In particular, the knob 36 comprises on an inner wall thereof facing the intermediate body 37, an inner hollow 67 sized for housing with limited clearance the terminal section 66 of the manoeuvre arm 64.

A flat recess 70, having circular sector shape of predetermined angular width and limited at the ends by two stroke end steps 72, is further made on the upper wall 37a of the intermediate body 37 and it houses the manoeuvre arm 64. The flat recess 70 forms, with the overhanging structure 35, a slit 71 of minimum space.

The angular shift in a rotation direction, or in the other, of the knob 36 thus substantially determines a traverse movement of the manoeuvre arm 64 in the flat recess 70.

According to a further embodiment, the knob 36 does not co-operate with the terminal section 66 and this latter can be manoeuvred directly by the user.

The gas interception in the device 10 according to the invention is performed in the following way.

By manoeuvring the knob 36 in rotation in one direction, for example anticlockwise, one acts by means of the arm 64 on the rotary element 40, selectively bringing it from an opening position, wherein the radial section 44 of the gas passage 42 is in gas communication with the horizontal section 12b of the gas passage 12, to a closing position, wherein the radial section 44 is angularly disjointed from the gas path 12.

It is to be noted that, in the opening position, the second tight ring 58 prevents the gas leaks from the valve body 11, and it ensures the tight passage thereof in the radial section 44.

In the closing position, instead, the side wall 40a of the rotary element 40 interrupts the gas path 12 and it adheres against the second ring 58 for ensuring and absolute tight interception of the gas.

The tight ring 58 thus has the double function on one side, in the opening position, of preventing the gas leaks from the valve body 11 and, on the other side, in the closed position, of ensuring the interception of gas.

A gas interception valve 25 is thus obtained of high construction simplicity and wherein, at the same time, a gas tight closing is ensured in time thanks to the co-operation between the tight ring 58 and the side wall 40a of the rotary element 40.

The two opening and closing positions, angularly shifted one with the other, preferably correspond with the two opposite stroke end positions of the arm 64 against the respective step 72 of the flat recess 70.

In this way, a user can easily know the two extreme opening and closing positions, simply by detecting the beating of the arm 64 against the step 72.

Preferably, on the upper face 36a of the knob 36 directional indicators are marked indicating the opening, and respectively closing, positions, of the rotary valve 25.

A front synoptic is further provided so as to have the immediate vision of the opening or closing state of the valve 25 simply by leaving the indication ON or OFF uncovered by a sector of the activating knob 36 of the manoeuvre arm 64.

The main advantage of the present invention stays in that it provides a pressure reduction device for a gas cylinder, wherein the gas interception valve is of simple realisation and it comprises mechanical elements easy to be found on sale, and which do not require any precise manufacturing.

Possible spreads or gaps formed further to a little precise mechanical manufacturing can be in fact easily corrected by means of the use of spacers, of tight rings of various sizes or by gauging the sizes of the jacket.

Another advantage of the device according to the invention stays in that all the elements, which compose the rotary valve, can be easily substituted in case of wear or breakage.

A further advantage stays in the use of an anti-friction jacket, having the double function of facilitating the rotation of the rotary element and, at the same time, of supporting the second tight ring co-operating with the rotary element for ensuring the tight closing of the gas path.

Another advantage of the present invention stays in that the arm of the rotary element can be activated in rotation directly by the user, without necessarily requiring the action of the tubular knob.

The small sections facing directly the cylinder pressure allow the rotation of the rotary element by applying negligible forces.

## Claims

1. A pressure reduction device for a gas cylinder, of the type comprising a valve body (11, 35, 37) intended for being co-axially associated with the gas cylinder, a gas path (12) extended in the valve body (11, 35, 37) between an inlet (14) of gas at high pressure and an outlet (16) of gas at reduced pressure, as well as gas interception means (25), at least one pressure reduction valve (26, 28) and a flow-rate regulation valve (27), placed along the gas path (12), **characterised in that** said gas interception means comprise a rotary valve (25), wherein the rotary valve (25) comprises a seat (38) in the valve body (37) placed in communication with at least one section (12b) of the gas path (12), a rotary element (40) housed in the seat (38) and provided with a passage (42) for the gas and at least one jacket (54) inserted in the valve body (37) between the seat (38) and the rotary element (40), the rotary element (40) being angularly shiftable in the valve body (37) between an opening position wherein the passage (42) is in communication with the section (12b) of the gas path (12) and a closing position wherein the passage (42) is disjointed from the section (12b) of the gas path (12).

2. A device according to claim 1, **characterised in that** the rotary element (40) is angularly shiftable around the axis (X) of said valve body (11).

3. A device according to claim 2, **characterised in that** the passage (42) comprises a radial section (44) effective to communicate with the corresponding section (12b) of the gas path (12) and at least one axial section (46).

4. A device according to claim 3, **characterised in that** the axial section (46) and the radial section (44) of the passage (42) are connected with an L-shape.

5. A device according to claim 2, **characterised in that** the seat (38) and the rotary element (40) have a substantially cylindrical shape.

6. A device according to claim 1, **characterised in that** the jacket (54) is made of anti-friction metal, preferably of bronze.

7. A device according to claim 1, **characterised in that** it comprises a radial opening (56) made in the jacket (54) and a tight ring (58) in the radial opening (56), placed in correspondence with said section (12b) of the gas path (12).

8. A device according to claim 1, **characterised in that** it comprises an arch-like opening made in the jacket (54) for centring this latter.

9. A device according to claim 1, **characterised in that** the rotary valve (25) comprises a manoeuvre arm (64), radially extending from the rotary element (40) outwards from the valve body (37).

10. A device according to claim 9, **characterised in that** the manoeuvre arm (64) has an end (65) being integral with the rotary element (40) and it comprises, at the opposite end, a terminal section (66) projecting from the valve body (37).

11. A device according to claim 9, **characterised in that** it comprises a knob (36), co-axially associated with the valve body (11) and angularly shiftable around the valve body (11) for controlling the angular shift of the rotary element (40).

12. A device according to claim 11, **characterised in that** said knob (36) comprises an inner hollow (67), sized for housing, with limited clearance, the terminal section (66) of the manoeuvre arm (64).

13. A device according to claim 9, **characterised in that** it comprises a flat recess (70), having a circular sector shape of predetermined angular width, which is made in the valve body (37) for housing the manoeuvre arm (64) of the rotary element (40).

## Patentansprüche

1. Druckverminderungsvorrichtung für eine Gasflasche der Art umfassend einen Ventilkörper (11, 35, 37), der zur koaxialen Ausrichtung mit der Gasflasche gedacht ist, einen Gasweg (12), der sich in dem Ventilkörper (11, 35, 37) zwischen einem Einlass (14) des Gases mit hohem Druck und einem Auslasse (16) des Gases mit reduziertem Druck erstreckt, sowie Gasunterbrechungsmittel (25), mindestens ein Druckverminderungsventil (26, 28) und ein Durchflusseinstellungsventil (27), das entlang des Gasweges (12) angeordnet ist, **dadurch gekennzeichnet, dass** das Gasunterbrechungsmittel ein Drehventil (25) umfasst, wobei das Drehventil (25) einen Sitz (38) in dem Ventilkörper (37), der in Verbindung mit mindestens einem Abschnitt (12b) des Gasweges (12) angeordnet ist, ein Drehelement (40), welches in dem Sitz (38) aufgenommen ist und mit einem Durchgang (42) für das Gas versehen ist, und mindestens eine Umhüllung (54) umfasst, welche in dem Ventilkörper (37) zwischen dem Sitz (38) und dem Drehelement (40) eingefügt ist, wobei das Drehelement (40) bezüglich seiner Winkelstellung in dem Ventilkörper (37) zwischen einer geöffneten Stellung, in der der Durchgang (42) in Verbindung mit dem Abschnitt (12b) des Gasweges (12) sich befindet, und einer geschlossenen Stellung, in der der Durchgang (42) von dem Abschnitt (12b) des Gaspfades (12) getrennt ist, verlagerbar ist.

2. Vorrichtung nach Anspruch 1, **dadurch gekennzeichnet, dass** das Drehelement (40) um die Achse (X) des Ventilkörpers (11) herum winklig verlagerbar ist.

3. Vorrichtung nach Anspruch 2, **dadurch gekennzeichnet, dass** der Durchgang (42) einen radialen Abschnitt (44), der zur Verbindung mit dem entsprechenden Abschnitt (12b) des Gaspfades (12) geeignet ist, und mindestens einen axialen Abschnitt (46) umfasst.

4. Vorrichtung nach Anspruch 3, **dadurch gekennzeichnet, dass** der axiale Abschnitt (46) und der radiale Abschnitt (44) des Durchgangs (42) L-förmig miteinander verbunden sind.

5. Vorrichtung nach Anspruch 2, **dadurch gekennzeichnet, dass** der Sitz (38) und das Drehelement (40) eine im Wesentlichen zylindrische Form aufweisen.

6. Vorrichtung nach Anspruch 1, **dadurch gekennzeichnet, dass** die Umhüllung (54) aus einem reibungsarmen Metall, vorzugsweise Bronze, hergestellt ist.

7. Vorrichtung nach Anspruch 1, **dadurch gekennzeichnet, dass** sie eine radiale Öffnung (56), die in der Umhüllung (54) vorgesehen ist, und einen Dichtring (58) in der radialen Öffnung (56) umfasst, der in Übereinstimmung mit dem Abschnitt (12b) des Gasweges (12) angeordnet ist.

8. Vorrichtung nach Anspruch 1, **dadurch gekennzeichnet, dass** sie eine bogenförmige Öffnung in der Umhüllung (54) umfasst, um diese zu zentrieren.

9. Vorrichtung nach Anspruch 1, **dadurch gekennzeichnet**, das Drehventil (25) einen Stellarm (64) umfasst, der sich radial von dem Drehelement (40) nach außen von dem Ventilkörper (37) erstreckt.

10. Vorrichtung nach Anspruch 9, **dadurch gekennzeichnet, dass** der Stellarm (64) ein Ende (65) aufweist, der einstückig mit dem Drehelement (40) ausgeführt ist, und er am gegenüberliegenden Ende einen Endbereich (66) umfasst, der von dem Ventilkörper (37) hervorsteht.

11. Vorrichtung nach Anspruch 9, **dadurch gekennzeichnet, dass** es einen Knauf (36) umfasst, der koaxial mit dem Ventilkörper (11) angeordnet ist und im Winkel um den Ventilkörper (11) herum zur Regelung der Winkeleinstellung des Drehelements (40) verlagerbar ist.

12. Vorrichtung nach Anspruch 11, **dadurch gekennzeichnet, dass** der Knauf (36) eine innere Ausnehmung (67), die zur Aufnahme des Endabschnittes (66) des Stellarms (64) mit begrenztem Freiraum größenmäßig ausgestaltet ist.

13. Vorrichtung nach Anspruch 9, **dadurch gekennzeichnet, dass** es eine flache Aussparung (70) mit einer Kreisbogenform von vorbestimmter Winkelbreite umfasst, die in dem Ventilkörper (37) zur Aufnahme des Stellarms (64) des Drehelements (60) ausgeführt ist.

## Revendications

1. Un dispositif de réduction de pression pour une bouteille à gaz, du type comprenant un corps de soupape (11, 35, 37) prévu pour être associé coaxialement avec la bouteille à gaz, un chemin de gaz (12) s'étendant dans le corps de soupape (11, 35, 37) entre une entrée (14) de gaz à haute pression et une sortie (16) de gaz à pression réduite, ainsi que des moyens d'interception du gaz (25), au moins une soupape de réduction de pression (26, 28) et une soupape de régulation de débit (27), placées le long du chemin de gaz (12), **caractérisé en ce que** lesdits moyens d'interception du gaz comprennent une soupape rotative (25), dans laquelle la soupape rotative (25) comprend un siège (38) dans le corps de soupape (37) placé de manière à communiquer avec au moins une partie (12b) du chemin de gaz (12), un élément rotatif (40) logé dans le siège (38) et doté d'un passage (42) pour le gaz et au moins une chemise (54) insérée dans le corps de soupape (37) entre le siège (38) et l'élément rotatif (40), l'élément rotatif (40) pouvant se déplacer de manière angulaire dans le corps de soupape (37) entre une position d'ouverture dans laquelle le passage (42) communique avec la partie (12b) du chemin de gaz (12) et une position de fermeture dans laquelle le passage (42) est séparé de la partie (12b) du chemin de gaz (12).

2. Dispositif selon la revendication 1, **caractérisé en ce que** l'élément rotatif (40) peut se déplacer de manière angulaire autour de l'axe (X) dudit corps de soupape (11).

3. Dispositif selon la revendication 2, **caractérisé en ce que** le passage (42) comprend une partie radiale (44) efficace pour communiquer avec la partie correspondante (12b) du chemin de gaz (12) et au moins une partie axiale (46).

4. Dispositif selon la revendication 3, **caractérisé en ce que** la partie axiale (46) et la partie radiale (44) du passage (42) sont reliées avec une forme en L.

5. Dispositif selon la revendication 2, **caractérisé en ce que** le siège (38) et l'élément rotatif (40) ont une forme sensiblement cylindrique.

6. Dispositif selon la revendication 1, **caractérisé en ce que** la chemise (54) est constituée de métal antifriction, de préférence du bronze.

7. Dispositif selon la revendication 1, **caractérisé en ce qu'**il comprend une ouverture radiale (56) pratiquée dans la chemise (54) et une bague étanche (58) dans l'ouverture radiale (56), placée de manière à correspondre avec ladite partie (12b) du chemin de gaz (12).

8. Dispositif selon la revendication 1, **caractérisé en ce qu'**il comprend une ouverture en forme d'arc pratiquée dans la chemise (54) pour centrer cette dernière.

9. Dispositif selon la revendication 1; **caractérisé en ce que** la soupape rotative (25) comprend un bras de manoeuvre (64), s'étendant radialement à partir de l'élément rotatif (40) vers l'extérieur du corps de soupape (37).

10. Dispositif selon la revendication 9, **caractérisé en ce que** le bras de manoeuvre (64) possède une extrémité (65) solidaire de l'élément rotatif (40) et il comprend, à l'extrémité opposée, une partie terminale (66) faisant saillie à partir du corps de soupape (37).

11. Dispositif selon la revendication 9, **caractérisé en ce qu'**il comprend un bouton (36), associé coaxialement avec le corps de soupape (11) et pouvant se déplacer de manière angulaire autour du corps de soupape (11) pour contrôler le déplacement angulaire de l'élément rotatif (40).

12. Dispositif selon la revendication 11, **caractérisé en ce que** ledit bouton (36) comprend un creux interne (67), dont les dimensions sont prévues pour loger, avec un jeu réduit, la partie terminale (66) du bras de manoeuvre (64).

13. Dispositif selon la revendication 9, **caractérisé en ce qu'**il comprend un évidement plat (70), ayant une forme de secteur circulaire d'une largeur angulaire prédéterminée, qui est pratiqué dans le corps de soupape (37) pour loger le bras de manoeuvre (64) de l'élément rotatif (40).
